(19) Europäisches Patentamt
European Patent Office
Office européen des brevets

(11) **EP 1 322 585 B1**

(12) **EUROPÄISCHE PATENTSCHRIFT**

(45) Veröffentlichungstag und Bekanntmachung des Hinweises auf die Patenterteilung:
**16.01.2008 Patentblatt 2008/03**

(21) Anmeldenummer: **01969749.9**

(22) Anmeldetag: **20.09.2001**

(51) Int Cl.:
*B01J 23/88* (2006.01)     *B01J 37/08* (2006.01)
*C07C 45/35* (2006.01)     *C07C 51/25* (2006.01)
*F27B 9/24* (2006.01)

(86) Internationale Anmeldenummer:
**PCT/EP2001/010911**

(87) Internationale Veröffentlichungsnummer:
**WO 2002/024620 (28.03.2002 Gazette 2002/12)**

(54) **VERFAHREN ZUR HERSTELLUNG EINES MULTIMETALLOXID-KATALYSATORS, VERFAHREN ZUR HERSTELLUNG UNGESÄTTIGTER ALDEHYDE UND/ODER CARBONSÄUREN UND BANDCALZINIERVORRICHTUNG**

METHOD FOR PRODUCING A MULTI METAL OXIDE CATALYST, METHOD FOR PRODUCING UNSATURATED ALDEHYDES AND/OR CARBOXYLIC ACIDS AND BAND CALCINATION DEVICE

PROCEDE DE PRODUCTION D'UN CATALYSEUR A BASE D'OXYDE POLYMETALLIQUE, PROCEDE DE PRODUCTION D'ALDEHYDES ET/OU D'ACIDES CARBOXYLIQUES INSATURES ET DISPOSITIF DE CALCINATION A BANDE

(84) Benannte Vertragsstaaten:
**DE**

(30) Priorität: **21.09.2000 DE 10046957**

(43) Veröffentlichungstag der Anmeldung:
**02.07.2003 Patentblatt 2003/27**

(60) Teilanmeldung:
**07021039.8**

(73) Patentinhaber: **BASF Aktiengesellschaft**
**67056 Ludwigshafen (DE)**

(72) Erfinder:
• **ARNOLD, Heiko**
**218814 Nanjing (CN)**
• **UNVERRICHT, Signe**
**68169 Mannheim (DE)**
• **FELDER, Raimund**
**67434 Neustadt (DE)**
• **HARTH, Klaus**
**67317 Altleiningen (DE)**

• **MÜLLER-ENGEL, Klaus, Joachim**
**76297 Stutensee (DE)**

(74) Vertreter: **Kinzebach, Werner et al**
**Reitstötter, Kinzebach & Partner (GbR)**
**Patentanwälte,**
**Sternwartstrasse 4**
**81679 München (DE)**

(56) Entgegenhaltungen:
EP-A- 0 275 671     EP-A- 0 750 942
EP-A- 0 807 465     WO-A-01/36364
US-A- 4 330 429     US-A- 4 534 780
US-A- 4 719 332     US-A- 5 637 546

• **PATENT ABSTRACTS OF JAPAN vol. 1996, no. 08, 30. August 1996 (1996-08-30) & JP 08 086571 A (KAO CORP), 2. April 1996 (1996-04-02) in der Anmeldung erwähnt**

Bemerkungen:
Die Akte enthält technische Angaben, die nach dem Eingang der Anmeldung eingereicht wurden und die nicht in dieser Patentschrift enthalten sind.

EP 1 322 585 B1

## Beschreibung

Die vorliegende Erfindung betrifft ein Verfahren zur Herstellung eines Katalysators mit einer aktiven Phase aus einer Multimetalloxidmasse.

Während Acrylsäure und -ester noch gegen Ende der 60er Jahre zum überwiegenden Teil nach dem Reppe-Verfahren hergestellt wurden, überwiegt heute die Herstellung durch Gasphasen-Oxidation von Propen. Die Propen-Oxidation zu Acrylsäure kann einstufig oder auch zweistufig durchgeführt werden. Als Katalysatoren für die heterogen katalysierte Reaktion finden in der Regel Multimetalloxidmassen Anwendung, die im Allgemeinen Schwermetallmolybdate als Hauptbestandteil und verschiedene Element-Verbindungen als Promotoren enthalten. Die Oxidation von Propen verläuft in einem ersten Schritt zu Acrolein und in einem zweiten Schritt zu Acrylsäure. Da beide Oxidationsschritte sich in ihrer Kinetik unterscheiden können, führen einheitliche Prozessbedingungen und ein einziger Katalysator in der Regel nicht zu einer optimalen Selektivität. Daher wurden in neuerer Zeit bevorzugt Zweistufenprozesse mit optimaler Anpassung von Katalysator und Prozessvariablen entwickelt. Im Allgemeinen wird Propen in Gegenwart von molekularem Sauerstoff in der ersten Stufe in exothermer Reaktion in einem Festbett-Röhrenreaktor zu Acrolein oxidiert. Die Reaktionsprodukte werden direkt in den zweiten Reaktor geleitet und zu Acrylsäure weiter oxidiert. Die in der zweiten Stufe erhaltenen Reaktionsgase können kondensiert und die Acrylsäure daraus durch Extraktion und/oder Destillation isoliert werden.

Die Oxidation von Propen zu Acrolein und/oder Acrylsäure ist stark exotherm. Die mit dem heterogenen Katalysator gefüllten Rohre eines Festbett-Rohrreaktors sind daher von einem Kühlmedium, in der Regel einer Salzschmelze wie einer eutektischen Mischung von $KNO_3$ und $NaNO_2$, umgeben. Die Reaktionswärme wird durch die Wand der Katalysator-gefüllten Rohre an das Salzbad abgegeben. Trotz der Kühlung durch das Kühlmedium stellt sich über die Länge und/oder den Querschnitt der Katalysator-gefüllten Rohre keine einheitliche Temperatur ein. Es kommt zur Ausbildung überhitzter Bereiche, der sogenannten "Hot Spots". So ist die Propenkonzentration des Reaktionsgemisches in der Nähe der Eintrittsstelle der Katalysator-gefüllten Rohre hoch, während das Reaktionsgemisch in der Nähe der Austrittsstelle an Propen verarmt ist, was die Bildung überhitzter Bereiche in der Nähe der Eintrittsstelle begünstigt. Da mit steigender Temperatur im Allgemeinen die Reaktionsgeschwindigkeit steigt, führt eine inhomogene Temperaturverteilung zu einem zusätzlichen Umsatz in den wärmeren Bereichen, wobei die damit verbundene exotherme Wärmeentwicklung zu einer Verstärkung des Temperaturungleichgewichts führt.

Die Bildung überhitzter Bereiche in den Katalysator-gefüllten Rohren ist aus verschiedenen Gründen nachteilig. In den überhitzten Bereichen kommt es zu einer übermäßigen Oxidation über die gewünschte Oxidationsstufe hinaus. Ferner beeinflusst die übermä-ßige thermische Belastung die Katalysatorleistung und -lebensdauer. Die Bildung überhitzter Bereiche kann schließlich zu einem unkontrollierten Reaktionsverlauf führen, der ein explosionsartiges Durchgehen der Reaktion zur Folge haben kann. Die exzessive Bildung von überhitzten Bereichen ist besonders bei Katalysatoren auf Molybdän-Basis nachteilig, da Molybdän-Verbindungen zur Sublimation neigen.

Zur Vereinheitlichung des Temperaturprofils in den Katalysator-gefüllten Rohren ist bereits vorgeschlagen worden, in den Rohren Katalysatoren unterschiedlicher Aktivität einzusetzen, wobei die Katalysatoraktivität axial in Strömungsrichtung des Reaktionsgemisches zunimmt. Katalysatoren unterschiedlicher Aktivität können z. B. durch Variation der chemischen Zusammensetzung erhalten werden, vergleiche US 5,276,178 und DE 30 06 894. Andererseits lässt sich die Katalysatoraktivität auch durch Variation der Calzinationstemperatur bei der Katalysatorherstellung variieren, vergleiche WO 98/24746. Die US 5,198,581 empfiehlt, Katalysatoren unterschiedlicher Raumerfüllung zu verwenden, wobei die Raumerfüllung axial in Strömungsrichtung des Reaktionsgemisches abnimmt. Diese Vorschläge sind insofern nachteilig, als die Herstellung mehrerer Katalysatoren unterschiedlicher Aktivität und das strukturierte Einfüllen der Katalysatoren in die Reaktionsrohre aufwendig und zeitraubend sind.

Die US 4,438,217 empfiehlt die Verwendung bestimmter Katalysatorgeometrien zur Verringerung des Problems der Bildung überhitzter Bereiche. Die EP 0 807 465 beschreibt einen Katalysator mit definiertem Anteil an aktiver Phase, Teilchengröße des Katalysators und Calzinationstemperatur.

Die JP 08086571-A beschreibt ein Behandlungsgerät, das einen Bandförderer mit einem gasdurchlässigen Band, das innerhalb eines hohlzylindrischen Gehäuses umläuft, und eine Vorrichtung umfasst, mittels der von innerhalb der Bandumlaufbahn ein Gas zum Förderteil des Bandes geleitet werden kann.

Die US-A 5,637,546 beschreibt ein verfahren zur Herstellung Mo und V enthaltender katalytisch aktiver Multimetalloxidmassen, wobei ein Katalysatorvorläufer bei 300 bis 450 °C in einer Gasatmosphäre calziniert wird, die neben Inertgasen und/oder Dampf aus 0,5 bis 4 Vol.-% $O_2$ and, über die Calcinationsdauer gemittelt, 1 bis 8 Vol.-% $NH_3$ besteht. Es ist angegeben, dass sich besonders wohldefinierte Calzinationsbedingungen bei Verwendung eines Bandcalzinationsofens realisieren lassen.

Die EP-A 750 942 beschreibt einen teilchenförmigen Katalysator für Wirbel für Fließbett-Gasphasenoxidationen. Es ist angegeben, dass zur homogenen und gleichmäßigen Calzinierung die Fließbett-Calzination besonders geeignet ist.

Der Erfindung liegt die Aufgabe zugrunde, einen Katalysator mit einer aktiven Phase aus einer Multimetalloxidmasse und ein Verfahren zu seiner Herstellung bereit zu stellen, der beim Einsatz zur Herstellung von α,β-monoethylenisch ungesättigten Aldehyden und/oder Carbonsäuren eine weitergehende Optimierung des Gleichgewichts von Aktivität, Selektivität und Katalysatorlebensdauer erlaubt.

Es wurde gefunden, dass die vorstehend im Zusammenhang mit der Ausbildung von "Hot Spots" geschilderten Probleme bei Katalysatoren, die nach bekannten Verfahren hergestellt sind, durch Fluktuationen der Aktivität der einzelnen Katalysatorteilchen über eine gegebene Gesamtheit von Katalysatorteilchen, die in einem technischen Festbettrohrreaktor enthalten sind, überlagert und verstärkt werden. Die eingesetzten heterogenen Katalysatoren mit einer aktiven Phase aus einer Multimetalloxidmasse weisen in Abhängigkeit von ihrer chemischen Zusammensetzung und Herstellungsbedingungen einen Temperaturbereich auf, in dem Aktivität, Selektivität und Katalysatorlebensdauer optimal ausgeglichen sind. Wenn sich in einer Gesamtheit der Katalysatorteilchen in einem Reaktor Teilchen stark differierender Aktivitäten befinden, so wird ein Teil des Katalysators zwangsläufig bei einer Temperatur betrieben, die mehr oder weniger weit vom optimalen Temperaturbereich der jeweiligen Katalysatorteilchen abliegt. Dies ist mit Einbußen der Ausbeute, Selektivität und Katalysatorlebensdauer verbunden.

Mit der vorliegenden Erfindung wurde gefunden, dass der Einsatz einer Gesamtheit von Katalysatorteilchen mit einer weitgehend homogenen Aktivität, d. h. einer möglichst engen Aktivitätsverteilung, eine Optimierung der Herstellung von α,β-monoethylenisch ungesättigten Aldehyden und/oder Carbonsäuren hinsichtlich Ausbeute, Selektivität und Katalysatorlebensdauer gestattet. Üblicherweise sind in einem technischen Reaktor zur Herstellung von α,β-monoethylenisch ungesättigten Aldehyden und/oder Carbonsäuren Katalysatormengen von mehreren Tonnen enthalten. Der Stand der Technik gibt keinen Hinweis, wie über eine derart große Katalysatormenge eine möglichst enge Aktivitätsverteilung erreicht werden kann. Die üblicherweise zur Calzinierung herangezogenen Vorrichtungen, wie Muffelöfen oder Hordenöfen, erlauben entweder lediglich die Herstellung kleiner Katalysatormengen oder weisen, sofern sie über ein ausreichend großes Fassungsvermögen verfügen, eine unzureichende Temperaturkonstanz auf. Da, wie in den nachstehenden Beispielen und Vergleichsbeispielen gezeigt, bereits geringe Unterschiede der Calzinierungstemperatur zu stark schwankender Aktivität führen können, führt eine unzureichende Temperaturkonstanz bei der Calzinierung zu Katalysatorchargen mit stark fluktuierender Aktivität. Das Mischen einer großen Menge mit Katalysator fluktuierender Aktivität zur Aktivitätsvereinheitlichung weist wesentliche Nachteile auf. zum einen ist der Katalysator beim Mischvorgang mechanischer Beanspruchung ausgesetzt, die bei Schalenkatalysatoren zum Abrieb bzw. bei Extrudaten und Katalysatortabletten zum Bruch führt. Zum anderen erreicht man durch Vermischen lediglich eine Vereinheitlichung auf niedrigem Niveau, da auch beim Vermischen Aktivitätsunterschiede zwischen einzelnen Katalysatorteilchen erhalten bleiben.

Mit der Erfindung wurde ein Verfahren zur einfachen und kostengünstigen Herstellung großer Mengen eines Katalysators mit einer aktiven Phase aus einer Multimetalloxidmasse mit enger Aktivitätsverteilung gefunden.

Die Erfindung betrifft ein Verfahren zur Herstellung eines für die Gasphasenoxidation von organischen Verbindungen zu α,β-ungesättigten Aldehyden und/oder Carbonsäuren geeigneten Katalysators mit einer aktiven Phase aus einer Multimetalloxidmasse, bei dem man einen teilchenförmigen Katalysatorvorläufer herstellt, der Oxide und/oder in Oxide überführbare Verbindungen der von Sauerstoff verschiedenen, die Multimetalloxidmasse konstituierende Elemente enthält, und diesen durch Calzinierung in eine katalytisch aktive Form überführt, wobei man einen Strom des teilchenförmigen Katalysatorvorläufers zur Calzinierung mit im Wesentlichen konstanter Geschwindigkeit durch wenigstens eine Calzinierungszone führt und senkrecht zur Fortbewegungsrichtung des Katalysatorvorläufers in der Calzinierungszone einen Gasstrom durch den Strom des Katalysatorvorläufers leitet, wobei die maximale zeitliche Schwankung der Temperatur und die maximale lokale Temperaturdifferenz in der Calzinierungszone jeweils ≤ 5 °C betragen.

Erfindungsgemäß hergestellte Katalysatoren können sowohl als Vollkatalysatoren als auch als Trägerkatalysatoren vorliegen. Im Falle der Vollkatalysatoren besteht der Katalysator im Wesentlichen aus der Multimetalloxidmasse. Der entsprechende Katalysatorvorläufer besteht dabei im Wesentlichen aus einem innigen Gemisch von Oxiden und/oder in Oxide überführbare Verbindungen der von Sauerstoff verschiedenen, die Multimetalloxidmasse konstituierenden Elemente. Das Gemisch wird gegebenenfalls unter Verwendung üblicher Hilfsmittel, wie Gleitmittel und/oder Formhilfsmittel, wie Graphit oder Stearinsäure, oder Verstärkungsmittel, wie Mikrofasern aus Glas, Asbest, Siliciumcarbid oder Calciumtitanat, zur gewünschten Katalysatorgeometrie verdichtet, z. B. durch Tablettieren, Extrudieren oder Strangpressen. Die Vollkatalysatoren können beliebige Geometrie, wie Zylinder, Kugeln usw. aufweisen; bevorzugte Katalysatorgeometrien sind Hohlzylinder, z. B. mit einem Außendurchmesser und einer Länge von 2 bis 10 mm und einer Wandstärke von 1 bis 3 mm.

Um Katalysatorvorläufer für erfindungsgemäß hergestellte Trägerkatalysatoren zu erhalten, werden zweckmäßigerweise Trägerkörper mit einem in der Regel pulverförmigen innigen Gemisch von Oxiden und/oder in Oxide überführbaren Verbindungen der von Sauerstoff verschiedenen, die Multimetalloxidmasse konstituierenden Elemente beschichtet. Zweckmäßigerweise kann zur Beschichtung der Trägerkörper die aufzubringende Pulvermasse befeuchtet und nach dem Aufbringen, z. B. mittels heißer Luft, wieder getrocknet werden. Die Schichtdicke der auf den Trägerkörper aufgebrachten Pulvermasse wird zweckmäßigerweise im Bereich von 50 bis 500 μm, vorzugsweise im Bereich von 100 bis 350 μm gewählt. Als Trägermaterialien können dabei übliche poröse oder unporöse Aluminiumoxide, Siliciumdioxid, Thoriumdioxid, Zirkondioxid, Siliciumcarbid oder Silicate, wie Magnesium- oder Aluminiumsilicat, verwendet werden.

Die Trägerkörper können regelmäßig oder unregelmäßig geformt sein, wobei regelmäßig geformte Trägerkörper mit vorzugsweise deutlich ausgebildeter Oberflächenrauhigkeit, z. B. Kugeln oder Hohlzylinder, bevorzugt sind.

Die Multimetalloxidmasse kann amorph und/oder kristallin vorliegen. Sie kann Kristallite eines definierten Kristallstrukturtyps oder Gemische derartiger Kristallite enthalten. Die Multimetalloxidmasse kann eine im Wesentlichen einheitliche Gemischzusammensetzung aufweisen oder Bereiche einer dispergierten Phase enthalten, deren chemische Zusammensetzung von der ihrer lokalen Umgebung verschieden ist. Dies ist z. B. der Fall bei sogenannten Schlüssel- oder Promotorphasen, die in einer Wirtsphase dispergiert sind. Die Bereiche abweichender chemischer Zusammensetzung weisen vorzugsweise einen Größtdurchmesser von 1 bis 25 $\mu$m, insbesondere 1 bis 20 und besonders bevorzugt 5 bis 15 $\mu$m, auf, wobei als Größtdurchmesser die längste durch den Schwerpunkt eines Partikels gehende Verbindungsstrecke zweier auf der Oberfläche des Partikels befindlichen Punkte angesehen wird. In der Regel wird die dispergierte Phase in feinteiliger Form getrennt vorab hergestellt und mit Oxiden und/oder zu Oxiden zersetzbaren Verbindungen der von Sauerstoff verschiedenen, die Wirtsphase der Multimetalloxidmasse konstituierenden Elemente vermischt.

Als Verbindungen, die in der Regel durch Erhitzen, gegebenenfalls in Anwesenheit von Sauerstoff, in Oxide überführbar sind, kommen vor allem Halogenide, Nitrate, Formiate, Oxalate, Citrate, Acetate, Carbonate, Aminkomplexsalze, Ammoniumsalze und/oder Hydroxide in Betracht. Verbindungen, wie Ammoniumhydroxid, Ammoniumcarbonat, Ammoniumnitrat, Ammoniumformiat, Ammoniumacetat, Ammoniumoxalat oder Essigsäure, die beim Calzinieren im Wesentlichen vollständig zu gasförmig entweichenden Verbindungen zerfallen und/oder zersetzt werden können, können als Porenbildner zusätzlich eingearbeitet werden. Die Oxide und/oder in Oxide überführbare Verbindungen der von Sauerstoff verschiedenen, die Multimetalloxidmasse konstituierenden Elemente werden vorzugsweise innig vermischt. Das innige Vermischen kann in trockener oder in nasser Form erfolgen. Erfolgt es in trockener Form, so werden die Ausgangsverbindungen zweckmäßigerweise als feinteilige Pulver eingesetzt. Vorzugsweise erfolgt das innige Vermischen jedoch in nasser Form. Üblicherweise werden dabei die Ausgangsverbindungen in Form einer wässrigen Lösung und/oder Suspension miteinander vermischt. Anschließend wird die erhaltene wässrige Masse getrocknet, wobei der Trocknungsprozess vorzugsweise durch Sprühtrocknung der wässrigen Mischung erfolgt.

Katalysatoren mit einer aktiven Phase aus einer Multimetalloxidmasse zur Herstellung von $\alpha,\beta$-monoethylenisch ungesättigten Aldehyden und/oder Carbonsäuren durch Gasphasenoxidation eines Alkans, Alkanols, Alkens und/oder Alkenals mit 3 bis 6 Kohlenstoffatomen sind an sich bekannt und z. B. in der EP 0 000 835, EP 0 575 897, DE 198 55 913, US 5,276,178, DE 30 06 894, US 5,198,581, WO 98/24746, US 4,438,217 und EP 0 807 465 beschrieben.

Diese Katalysatoren sind besonders zur Herstellung von $\alpha,\beta$-monoethylenisch ungesättigten Aldehyden und/oder Carbonsäuren in Hochlastfahrweise geeignet.

Die Multimetalloxidmassen enthalten vorzugsweise wenigstens ein unter Molybdän und Wolfram ausgewähltes erstes Metall und wenigstens ein unter Wismut, Tellur, Antimon, Zinn, Kupfer, Eisen, Cobalt und/oder Nickel ausgewähltes zweites Metall.

Besonders bevorzugte Multimetalloxidmassen weisen die Formel I oder II auf,

$$[X^1_a X^2_b O_x]_p [X^3_c X^4_d X^5_e X^6_f X^7_g X^2_h O_y]_q \qquad (I)$$

$$Mo_{12} Bi_i X^8_k Fe_l X^9_m X^{10}_n O_z \qquad (II)$$

worin bedeuten

$X^1$      Wismut, Tellur, Antimon, Zinn und/oder Kupfer; vorzugsweise Wismut;

$X^2$      Molybdän und/oder Wolfram,

$X^3$      ein Alkalimetall, Thallium und/oder Samarium; vorzugsweise Kalium;

$X^4$      ein Erdalkalimetall, Nickel, Cobalt, Kupfer, Mangan, Zink, Zinn, Cadmium und/oder Quecksilber; vorzugsweise Nickel und/oder Cobalt;

$X^5$      Eisen, Chrom, Cer und/oder Vanadium; vorzugsweise Eisen;

$X^6$      Phosphor, Arsen, Bor und/oder Antimon,

$X^7$      ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran; vorzugsweise Silicium, Aluminium, Titan und/oder Zirkonium;

a    0,01 bis 8,

b    0,1 bis 30,

c    0 bis 4,

d    0 bis 20,

e    0 bis 20,

f    0 bis 6,

g    0 bis 15,

h    8 bis 16,

x,y    Zahlen, die durch die Wertigkeit und Häufigkeit der von Sauerstoff verschiedenen Elemente in I bestimmt sind,

p,q    Zahlen, deren Verhältnis p/q 0,1 bis 10 beträgt,

$X^8$    Cobalt und/oder Nickel; vorzugsweise Cobalt;

$X^9$    Silicium und/oder Aluminium; vorzugsweise Silicium;

$X^{10}$    ein Alkalimetall, vorzugsweise Kalium, Natrium, Cäsium und/oder Rubidium; insbesondere Kalium;

i    0, 1 bis 2,

k    2 bis 10,

l    0,5 bis 10,

m    0 bis 10,

n    0 bis 0,5,

z    Zahl, die durch die Wertigkeit und Häufigkeit der von Sauerstoff verschiedenen Elemente in II bestimmt ist.

Das erfindungsgemäße Verfahren ist besonders geeignet zur Herstellung von Katalysatoren mit einer aktiven Phase aus einer Multimetalloxidmasse der Formel II, da diese Massen eine besonders ausgeprägte Abhängigkeit der Aktivität von der Calzinierungstemperatur aufweisen.

Multimetalloxidmassen der Formel I sind an sich aus der EP 0 000 835 und EP 0 575 897, Multimetalloxidmassen der Formel II an sich aus der DE 198 55 913 bekannt.

Multimetalloxidmassen der Formel I enthalten dreidimensional ausgedehnte, abgegrenzte Bereiche der chemischen Zusammensetzung $X^1_a X^2_b O_x$, deren chemische Zusammensetzung von der ihrer lokalen Umgebung verschieden ist. Der Größtdurchmesser der abgegrenzten Bereiche beträgt vorzugsweise 1 bis 25 $\mu$m, insbesondere 1 bis 20 $\mu$m und besonders bevorzugt 5 bis 15 $\mu$m. Katalysatorvorläufer für Katalysatoren mit einer aktiven Phase aus einer Multimetalloxidmasse der Formel I werden geeigneterweise dadurch erhalten, dass man zunächst ein calziniertes Oxid $X^1_a X^2_b O_x$ vorbildet, indem man z. B. wasserlösliche Salze von $X^1$ mit Oxosäuren oder deren Ammoniumsalzen von $X^2$ in wässriger Lösung mischt, die Lösung trocknet und die getrocknete Masse calziniert, das erhaltene Oxid gegebenenfalls zerkleinert und Teilchen einer gewünschten Kornklasse abtrennt. Das getrennt vorgebildete Oxid wird dann mit Oxiden und/oder in Oxide überführbaren Verbindungen von $X^3$, $X^4$, $X^5$, $X^6$, $X^7$ und $X^2$ in gewünschten stöchiometrischen Verhältnissen vermischt und, falls das Vermischen im nassen Zustand erfolgt, getrocknet.

Katalysatorvorläufer für Katalysatoren mit einer aktiven Phase aus einer Multimetalloxidmasse der Formel II werden geeigneterweise erhalten, indem man eine erste wässrige Lösung von Verbindungen der Elemente Wismut, Eisen und $X^8$ herstellt, eine zweite wässrige Lösung von Verbindungen der Elemente Molybdän und $X^{10}$ herstellt, die erste und zweite wässrige Lösung vermischt, wobei man die erste oder die zweite wässrige Lösung oder deren Gemisch mit einer Lösung oder Suspension einer Verbindung des Elementes $X^9$ vermischt, und das erhaltene Gemisch oder Fällungsprodukt vorzugsweise durch Sprühtrocknung trocknet.

Zur abschließenden Calzinierung wird ein Strom des teilchenförmigen Katalysatorvorläufers, vorzugsweise ein zeitlich im Wesentlichen konstanter Massenstrom, mit im Wesentlichen konstanter Geschwindigkeit durch wenigstens eine Calzinierungszone geführt.

Der Begriff "Calzinierung" soll vorliegend auch der eigentlichen Calzinierung vorgelagerte thermische Behandlungsschritte, wie eine abschließende Trocknung und/oder Zersetzung, umfassen. Zweckmäßigerweise führt man den Strom des teilchenförmigen Katalysatorvorläufers nacheinander durch wenigstens zwei, z. B. zwei bis zehn, Calzinierungszonen, die in der Regel auf unterschiedliche Temperatur thermostatiert sind. Auf diese Weise lassen sich unterschiedliche Temperaturprofile verwirklichen, die von dem Strom des teilchenförmigen Katalysatorvorläufers durchlaufen werden. So können die aufeinander folgenden Calzinierungszonen z. B. auf stufenweise steigende Temperaturen thermostatiert sein. Die einzelnen Calzinierungszonen können unterschiedliche räumliche Ausdehnung aufweisen, so dass bei konstanter Geschwindigkeit des Stroms des teilchenförmigen Katalysatorvorläufers unterschiedliche Verweilzeiten in den einzelnen Calzinierungszonen resultieren.

In wenigstens einer Calzinierungszone wird der Katalysatorvorläufer auf eine Temperatur von im Allgemeinen 400 bis 600 °C, vorzugsweise 450 bis 530 °C erwärmt. Bei Ausübung des erfindungsgemä-ßen Verfahrens unter Verwendung von acht Calzinierungszonen ist z. B. folgendes Temperaturprofil geeignet:

1: 100-200 °C; 2: 150-250 °C; 3: 200-300 °C; 4: 250-350 °C;
5: 350-400 °C; 6: 400-550 °C; 7: 400-550 °C; 8: 400-550 °C.

Bei Verwendung von drei Zonen ist folgendes Temperaturprofil geeignet:

1: 100-400 °C; 2: 250-550 °C; 3: 400-550 °C.

Bei Verwendung von vier Zonen ist folgendes Temperaturprofil geeignet:

1: 100-250 °C; 2: 200-350 °C;
3: 350-550 °C; 4: 400-550 °C.

Bei Verwendung von sechs Zonen ist folgendes Temperaturprofil geeignet:

1 : 100-200 °C; 2: 150-300 °C; 3: 200-350 °C;
4: 300-400 °C; 5: 400-500 °C; 6: 400-600 °C.

Bei Verwendung von 12 Zonen ist folgendes Temperaturprofil geeignet:

1: 100-200 °C; 2: 150-250 °C; 3: 200-300 °C; 4: 250-350 °C;
5: 350-400 °C; 6: 400-500 °C; 7: 400-550 °C; 8: 400-550 °C;
9: 400-600 °C; 10: 400-600 °C; 11: 400-700 °C; 12: 400-700 °C.

Für die erfolgreiche Ausübung des erfindungsgemäßen Verfahrens ist es wesentlich, dass die Temperatur in der Calzinierungszone (in den Calzinierungszonen) zeitlich und lokal im Wesentlichen konstant ist. Die Temperaturkonstanz kann mittels Temperaturmesseinrichtungen, d. h. Thermoelementen, überprüft werden. Pro Calzinierungszone sind vorzugsweise wenigstens 4, insbesondere wenigstens 6 und besonders bevorzugt wenigstens 10 Elemente, vorgesehen, die möglichst äquidistant über die Calzinierungszone angeordnet sind. Vorzugsweise weicht der Mittelwert der Stundenmittelwerte der an den einzelnen Thermoelementen einer Calzinierungszone gemessenen Temperatur maximal um 5 °C, insbesondere maximal 3°C, besonders bevorzugt maximal 2 °C, vom jeweiligen Sollwert ab. Die Aufzeichnung der an den einzelnen Thermoelementen gemessenen Temperaturen und die Bildung der Mittelwerte erfolgen zweckmäßigerweise automatisiert durch einen entsprechend programmierten Computer. Durch diesen erfolgt mit Vorteil auch die Regelung bzw. Steuerung der Beheizung der Calzinierungszonen. Die Thermoelemente werden mit Vorteil regelmäßig kalibriert, um zu gewährleisten, dass die maximale Abweichung der gemessenen Temperatur von der tatsächlichen Temperatur vorzugsweise weniger als 0,5 °C beträgt.

Die maximale zeitliche Schwankung der Temperatur in der Calzinierungszone (in den Calzinierungszonen) beträgt ≤ 5 °C, insbesondere ≤ 3 °C, besonders bevorzugt ≤ 2 °C. Als zeitliche Schwankung der Temperatur wird die Standardabweichung der an einem einzelnen Thermoelement einer Calzinierungszone über eine Stunde gemessenen Temperatur angesehen. Die maximale lokale Temperaturdifferenz, d. h. die maximale Temperaturdifferenz innerhalb der Calzinierungszone (in den Calzinierungszonen) beträgt ≤ 5 °C, insbesondere ≤ 3 °C, besonders bevorzugt ≤ 2 °C. Als lokale Temperaturdifferenz wird die Standardabweichung der Stundenmittelwerte der an den Thermoelementen einer Calzinierungszone gemessenen Temperaturen angesehen.

Die Atmosphäre in der Calzinierungszone kann aus Inertgas, z. B. Stickstoff oder Argon, einem Gemisch aus Inertgas und Sauerstoff, z. B. Luft, reduzierend wirkenden Gasen, wie Kohlenwasserstoffen, z. B. Methan, Aldehyden, z. B. Acrolein oder Ammoniak, oder auch aus einem Gemisch aus Sauerstoff und reduzierend wirkenden Gasen bestehen. Bei einer Calzinierung unter reduzierenden Bedingungen ist allerdings zu beachten, dass die metallischen Konstituenten nicht bis zum Element reduziert werden. Zweckmäßigerweise liegt in der Calzinierungszone daher ein Sauerstoff enthaltendes Gas, wie insbesondere Luft, vor. Zur Vergleichmäßigung der Temperatur in der Calzinierungszone und/oder zum Abtransport gegebenenfalls entstehender Zersetzungsgase leitet man senkrecht zur Fortbewegungsrichtung des Katalysatorvorläufers in der Calzinierungszone einen Strom eines Gases über den Katalysatorvorläufer. Geeignete Gase sind die vorstehend genannten, insbesondere Luft. Insbesondere zur Herstellung von Katalysatoren mit einer Molybdän und Wismut enthaltenden Multimetalloxidmasse kann es vorteilhaft sein, die Calzinierung in einer Atmosphäre durchzuführen, die 1 Vol-% oder mehr Stickoxid und 0,5 Vol-% oder mehr Sauerstoff enthält, wie in der EP-A-558 028 beschrieben.

Zur Durchführung des erfindungsgemäßen Verfahrens verwendet man zweckmäßigerweise eine Bandcalziniervorrichtung mit wenigstens einer beheizbaren Kammer und einem die Kammer durchlaufenden Förderband zur Aufnahme des teilchenförmigen Katalysatorvorläufers. Bei dem Förderband handelt es sich in der Regel um ein Endlosband, das horizontal durch die beheizbare Kammer fortbewegbar ist und an seinem einen Ende eine ansteigende Umkehrbahn und an seinem anderen Ende eine absteigende Umkehrbahn bildet. Vielfach ist es vorteilhaft, ein Gehäuse vorzusehen, innerhalb dessen das Förderband umläuft, d. h. das die Kammer(n) und die an- und absteigende Umkehrbahn umgibt. In der Nähe der ansteigenden Umkehrbahn sind zweckmäßigerweise Mittel zum Aufgeben des teilchenförmigen Katalysatorvorläufers auf das Förderband und an der absteigenden Umkehrbahn zweckmäßigerweise Mittel zur Entnahme des calzinierten Katalysators vom Förderband vorgesehen. Die Mittel zum Aufgeben bestehen z. B. aus einem mit dem teilchenförmigen Katalysatorvorläufer gefüllten Schacht, wobei durch die Bewegung des Förderbands eine Schüttung des Katalysatorvorläufers mit im Wesentlichen konstanter Schichtdicke unter dem Schacht weggezogen wird. Der Schacht weist zweckmäßigerweise eine Abstreifvorrichtung, z. B. ein verstellbares Wehr, auf. An der absteigenden Umkehrbahn wird der calzinierte Katalysator vom Förderband abgeworfen und zweckmäßigerweise durch geeignete Mittel gesammelt. Da in der Calzinierungszone ein Gas über den Katalysatorvorläufer geleitet wird, ist das Förderband gasdurchlässig und besteht z. B. aus einem perforierten Band oder Gewebe oder Gewirk aus Metalldraht oder einem anderen hitzebeständigen Material. Die Geschwindigkeit des Förderbands beträgt in der Regel etwa 0,1 bis 5 cm/min. Um den teilchenförmigen Katalysatorvorläufer nacheinander durch mehrere Calzinierungszonen unterschiedlicher Temperatur zu leiten, verwendet man zweckmäßigerweise eine Bandcalziniervorrichtung, die wenigstens zwei, z. B. zwei bis zehn, beheizbare Kammern aufweist, die auf unterschiedliche Temperatur regelbar sind.

Die Beheizung der Kammer(n) kann auf verschiedene Weise erfolgen, etwa durch elektrische Beheizung über z. B. in der Kammerwand eingebaute Widerstandselemente. Vorzugsweise erfolgt die Beheizung jedoch indirekt, d. h. durch Wärmequellen, wie Brenner, die außerhalb der Kammer(n) geeigneterweise unterhalb der Kammer(n), angeordnet sind. Die Brenner werden mit einem brennbaren Gas, wie Erdgas, betrieben.

In der Kammer (den Kammern) der Bandcalziniervorrichtung wird die Calzinierungszone durch die Breite der Auflagefläche des Förderbands sowie die maximale Schütthöhe des teilchenförmigen Katalysatorvorläufers auf dem Förderband definiert. Die zur Überwachung der Temperaturkonstanz in der Calzinierungszone vorgesehenen Thermoelemente sind vorzugsweise gleichmäßig über die Auflagefläche des Förderbandes und die Schütthöhe verteilt. Zur Erzielung einer möglichst hohen örtlichen Temperaturkonstanz in der Calzinierungszone wird die Atmosphäre in den beheizbaren Kammern vorzugsweise umgewälzt. Eine speziell zur Durchführung des erfindungsgemäßen Verfahrens konzipierte Bandcalziniervorrichtung weist daher in der Kammer Mittel zum Erzeugen einer Gaszirkulation aufgrund erzwungener Konvektion auf.

In einer bevorzugten Ausführungsform umfassen die Mittel zur Erzeugung der Gaszirkulation einen Ventilator, der geeigneterweise oberhalb des Förderbands in der Kammer (den Kammern) angeordnet ist. In geeigneten Ausführungsformen umfassen die Mittel zum Erzeugen der Gaszirkulation außerdem Gasleiteinrichtungen zum Leiten der Gaszirkulation innerhalb der Kammer, wobei sich die Gasleiteinrichtungen innerhalb der Kammer jeweils am Rand des Förderbands im Wesentlichen in einer Ebene senkrecht zur Auflagefläche des Förderbands erstrecken. Die Mittel zum Erzeugen der Gaszirkulation und/oder die Gasleiteinrichtungen sind zweckmäßigerweise so ausgebildet, dass das Gas durch das gasdurchlässige Förderband und die darauf befindliche Schüttung des teilchenförmigen Katalysatorvorläufers aufsteigt und an den Wänden der Kammer wieder absteigt. Andererseits ist aber auch eine Gaszirkulation in umgekehrter Richtung vorstellbar. Weist die Bandcalziniervorrichtung wenigstens zwei beheizbare Kammern auf, sind diese vorzugsweise so gegeneinander abgegrenzt, dass im Wesentlichen kein Gasaustausch zwischen den Kammern stattfindet. Zur Entfernung von Zersetzungsgasen und dergleichen wird vorzugsweise kontinuierlich oder periodisch ein Teil des in der Kammer umgewälzten Gases entfernt und durch Frischgas ersetzt. Die Zufuhr von Frischgas wird dabei so gesteuert, dass die Temperaturkonstanz in der Kammer nicht beeinträchtigt wird. Das Volumen des pro Zeiteinheit in der Kammer zirkulierten Gases ist in der Regel größer als das Volumen des der Kammer pro Zeiteinheit zu- oder daraus abgeführten Gases und beträgt vorzugsweise wenigstens das Fünffache davon.

Gewünschtenfalls können auch mehrere, z. B. zwei oder drei, der oben beschriebenen Bandcalziniervorrichtungen nacheinander durchlaufen werden, um z. B. durch Variation der Schütthöhe und/oder Verweilzeit eine unterschiedliche Wärmetönung einzelner Schritte zu optimieren. Der Katalysatorvorläufer kann gegebenenfalls nach dem Durchlaufen einer Vorrichtung und vor dem Durchlaufen einer weiteren Vorrichtung gesammelt und zwischengelagert werden.

Durch das erfindungsgemäße Verfahren können große Mengen eines Katalysators, der zur Herstellung von $\alpha,\beta$-monoethylenisch ungesättigten Aldehyden und/oder Carbonsäuren durch Gasphasenoxidation von organischen Verbindungen, wie z. B. Alkanen, Alkanolen, Alkenen und/oder Alkenalen mit 3 bis 6 Kohlenstoffatomen, geeignet ist und eine aktive Phase einer Multimetalloxidmasse aufweist, mit enger Aktivitätsverteilung innerhalb der hergestellten Charge erhalten werden. Für die Zwecke der vorliegenden Erfindung ist die Aktivität des Katalysators als die Temperatur definiert, bei der ein Propenumsatz von 95 % erhalten wird, wenn ein Gemisch aus 5 Vol.-% Propen, 9,5 Vol.-% Sauerstoff und 85,5 Vol.-% Stickstoff mit 100 Nl/h (Volumen im Normzustand bei 0 °C und 1013 mbar) über 100 g Katalysator geleitet wird. Zur Bestimmung ist ein Versuchsaufbau geeignet, wie er weiter unten beschrieben ist.

Erfindungsgemäß gelingt die Herstellung von Chargen eines Katalysators, der als aktive Phase eine Multimetalloxidmasse der vorstehend angegebenen Formel I oder II aufweist, von wenigstens 100 kg, insbesondere wenigstens 300 kg, besonders bevorzugt wenigstens 1 t (Tonne), wobei die Standardabweichung der wie oben definierten Aktivität beliebig herausgegriffener Stichproben weniger als 7 °C, insbesondere weniger als 3,5 °C und besonders bevorzugt weniger als 2,5 °C beträgt.

Die Anzahl der Stichproben beträgt wenigstens 5, insbesondere wenigstens 10, z. B. wenigstens eine Stichprobe pro 100 kg Produktionsmenge. Der Umfang einer Stichprobe beträgt vorzugsweise etwa 1 kg, woraus nach Homogenisierung 100 g zur oben angegebenen Aktivitätsbestimmung entnommen werden. Im besonders bevorzugten Fall lässt sich der Mittelwert der Aktivität bei Herstellung von 1000 kg Produkt und Entnahme einer Stichprobe aus jedem 100 kg-Gebinde und einer nachfolgenden Homogenisierung der Stichproben mit einer Zuverlässigkeit von 95 % auf $\pm$ 1,6 °C abschätzen.

Die Katalysatorcharge kann zur Lagerung oder zum Transport geeigneterweise in Behälter aus Kunststoff oder Blech abgefüllt werden, die ein Fassungsvermögen von z. B. 50 l oder 100 l aufweisen. In vielen Fällen ist es zweckmäßig, zur oben angesprochenen Stichprobenentnahme eine Probe von z. B. 1 kg pro Behälter zu entnehmen.

Erfindungsgemäß hergestellte Katalysatoren eignen sich besonders zur Herstellung von $\alpha,\beta$-monoethylenisch ungesättigten Aldehyden und/oder Carbonsäuren durch Gasphasenoxidation von Alkanen, Alkanolen, Alkenen und/ oder Alkenalen mit 3 bis 6 Kohlenstoffatomen. Sie eignen sich ferner zur Herstellung von Nitrilen durch Ammonoxidation, insbesondere von Propen zu Acrylnitril und von 2-Methylpropen bzw. tert-Butanol zu Methacrylnitril. Sie eignen sich ferner zur oxidativen Dehydrierung organischer Verbindungen.

Die erfindungsgemäß hergestellten Katalysatoren eignen sich insbesondere zur Herstellung von Acrolein, Acrylsäure, Methacrolein und Methacrylsäure, wobei als Ausgangsverbindungen vorzugsweise Propen oder 2-Methylpropen, tert-Butanol bzw. Methacrolein eingesetzt werden. Besonders eignen sich erfindungsgemäß hergestellte Katalysatoren zur Herstellung von Acrolein aus Propen. Als Oxidationsmittel wird in an sich bekannter Weise Sauerstoff, zweckmäßigerweise durch inerte Gase verdünnt, eingesetzt. Als inerte Gase kommen z. B. Stickstoff oder Wasserdampf in Betracht. Geeignete Reaktionstemperaturen und Reaktionsdrücke sind dem Fachmann bekannt, wobei als allgemeiner Rahmen eine Temperatur von 250 bis 450 °C und ein Druck von 0,5 bis 4 bar (Überdruck) angegeben werden kann.

Erfindungsgemäß hergestellte Katalysatoren werden in einem Verfahren zur Herstellung von $\alpha,\beta$-monoethylenisch ungesättigten Aldehyden und/oder Carbonsäuren verwendet, bei dem man einen gasförmigen Strom eines Alkans, Alkanols, Alkens und/oder Alkenals mit 3 bis 6 Kohlenstoffatomen bei erhöhter Temperatur in Gegenwart von molekularem Sauerstoff durch wenigstens eine Reaktionszone leitet, die eine Charge eines Katalysators der vorstehend definierten, engen Aktivitätsverteilung enthält.

Die erfindungsgemäß hergestellten Katalysatoren eignen sich besonders für die Hochlastfahrweise. Die Belastung des Katalysators mit dem Alkan, Alkanol, Alken und/oder Alkenal beträgt dabei wenigstens 160 N1, insbesondere wenigstens 165 N1 und besonders bevorzugt wenigstens 170 N1 pro 1 Katalysator pro Stunde.

Mit Vorteil leitet man den gasförmigen Strom nacheinander durch eine erste und eine zweite Reaktionszone, wobei die Temperatur der ersten Reaktionszone 300 bis 330°C und die Temperatur der zweiten Reaktionszone 300 bis 365 °C beträgt und wenigstens 5 °C über der Temperatur der ersten Reaktionszone liegt. Die erste Reaktionszone erstreckt sich in der Regel bis zu einem Umsatz des Alkans, Alkanols, Alkens und/oder Alkenals von 40 bis 80 mol-%, vorzugsweise 50 bis 70 mol-%.

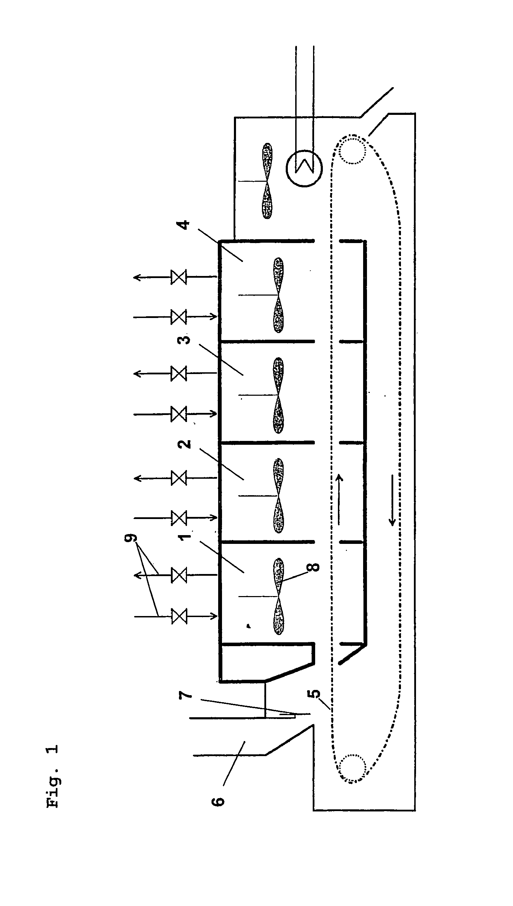

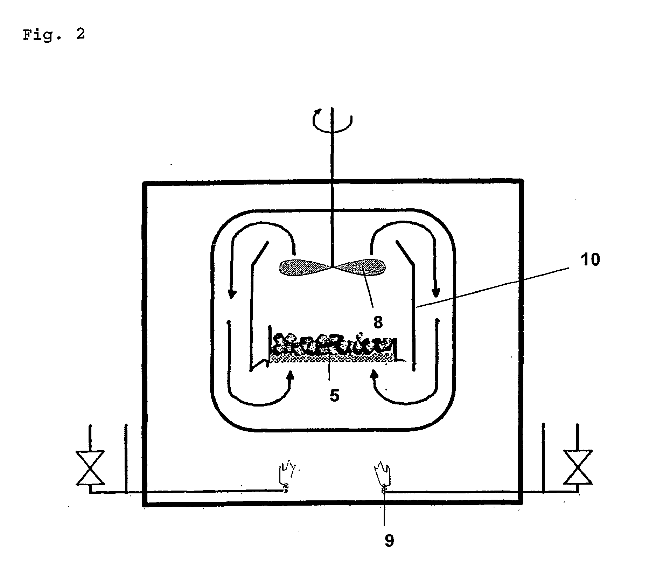

Die Erfindung wird nun durch die beiliegenden Figuren und die nachstehenden Beispiele näher veranschaulicht. Fig. 1 zeigt einen Längsschnitt, Fig. 2 einen Querschnitt durch eine erfindungsgemäß verwendbare Bandcalziniervorrichtung.

Mit Bezug auf Fig. 1 weist die dargestellte Calziniervorrichtung vier Kammern auf (1, 2, 3, 4), durch die ein Förderband (5) läuft. Die Vorrichtung weist einen Bunker (6) mit einem Wehr (7) auf. Oberhalb des Förderbandes (5) befinden sich in jeder Kammer Ventilatoren (8). Jede Kammer ist mit Zu- und Ablufteinrichtungen (9) versehen. Im Betrieb ist der Bunker (6) mit dem teilchenförmigen Katalysatorvorläufer gefüllt. Durch die Bewegung des Förderbandes (5) wird unter dem Wehr (7) eine Schicht des Katalysatorvorläufers mit konstanter Schütthöhe weggezogen und durchläuft nacheinander die Kammern der Calziniervorrichtung.

Mit Bezug auf Fig. 2 wird jede Kammer durch Brenner (9) beheizt. An den Rändern des Förderbands sind im Wesentlichen in einer Ebene senkrecht zur Auflagefläche des Förderbands Gasleitbleche (10) angeordnet, die zusammen mit den Ventilatoren (8) dafür sorgen, dass die umgewälzte Atmosphäre in jeder Kammer durch das gasdurchlässige Förderband (5) aufsteigt und an den Wänden der Kammer wieder absteigt.

Die in den Beispielen hergestellten Katalysatoren werden durch die Parameter Umsatz, Acrolein-Selektivität ($S_{ACR}$) und Acrylsäure-Selektivität ($S_{ACS}$) charakterisiert. Diese Parameter wurden wie folgt bestimmt:

In ein Stahlrohr mit einem Innendurchmesser von 15 mm und einem Außendurchmesser von 20 mm wurde zuerst eine 15 cm lange Schüttung aus Steatitkugeln eines Durchmessers von 2 bis 3 mm eingefüllt. Anschließend wurden 100 g des zu testenden Katalysators eingefüllt, wobei eine durchschnittliche Schütthöhe von 60 bis 70 cm resultierte. Das Stahlrohr wurde mittels eines Stickstoffdurchspülten Salzbades über die gesamte Länge der den Katalysator und die Steatitkugeln umfassenden Schüttung temperiert. Bei einer konstanten Feedgasmenge von 100 Nl/h (Volumen im Normzustand) einer Zusammensetzung von 5 Vol.-% Propen, 9,5 Vol.-% Sauerstoff und 85,5 Vol.-% Stickstoff wurde die Temperatur des Salzbades so lange variiert, bis der am Ausgang des Stahlrohrs gemessene Propen-Umsatz 95 % betrug. Der Propen-Umsatz ist wie folgt definiert:

$$U_{Propen} \ [\%] \ = \ \frac{C_{Propen, \ Ausgang}}{C_{Propen, \ Eingang}} \ \text{x} \ 100$$

Außerdem wurde die Selektivität zu den Wertprodukten Acrolein und Acrylsäure gemessen. Diese berechnen sich wie folgt:

$$S_{ACR} \ [\%] \ = \ \frac{C_{ACR, \ Ausgang}}{C_{Propen, \ Eingang} \ - \ C_{Propen, \ Ausgang}} \ \text{x} \ 100$$

$$S_{ACS} \ [\%] \ = \ \frac{C_{ACS, \ Ausgang}}{C_{Propen, \ Eingang} \ - \ C_{Propen, \ Ausgang}} \ \text{x} \ 100$$

Die Wertprodukt-Selektivität berechnet sich zu:

$$S_{WP} \ [\%] \ = \ S_{ACS} \ + \ S_{ACR}$$

Beispiel 1

Es wurde zunächst eine Ausgangsmasse der Wirtsphase hergestellt. Hierzu wurden bei 60 °C portionsweise 244 kg Ammoniumheptamolybdat in 660 l Wasser gelöst und unter Rühren mit 1,12 kg einer 47,5 gew.-%igen Kaliumhydroxidlösung von 20 °C versetzt. Es wurde eine zweite Lösung hergestellt, indem man zu 288,7 kg einer Cobaltnitrat-Lösung (12,5 Gew.-% Cobalt) 133,8 kg einer Eisennitratlösung (13,8 Gew.-% Eisen) gab, wobei die Temperatur auf 60 °C gehalten wurde. Die zweite Lösung wurde innerhalb eines Zeitraums von 30 Min. bei 60 °C zu der Molybdat-Lösung gegeben. 15 Min. nach beendeter Zugabe wurden 19,16 kg Kieselsol (Dichte 1,36-1,42 g/ml, pH 8,5 bis 9,5, Alkaligehalt maximal 0,5 Gew.-%; 46,80 Gew.-% $SiO_2$) in die erhaltene Maische gegeben. Danach rührte man 15 Min. Die erhaltene Maische wurde anschließend sprühgetrocknet, wobei ein Pulver mit einem Glühverlust (3 h bei 600 °C) von etwa 30 Gew.-% erhalten wurde.

Es wurde dann eine feinteilige Promotorphase hergestellt. Zu 775 kg einer salpetersauren Wismutnitrat-Lösung (freie Salpetersäure 3-5 %, Dichte 1,22-1,27 g/ml, 11,2 Gew.-% Wismut) wurden unter Rühren portionsweise 209,3 kg Wolframsäure (72,94 Gew.-% Wolfram) gegeben. Man rührte die erhaltene Maische 2 h lang und trocknete sie durch Sprühtrocknung. Man erhielt ein Pulver mit einem Glühverlust (3 h bei 600 °C) von 12 Gew.-%. Dieses Pulver wurde mit einer geringen Menge Wasser in einem Kneter angeteigt und mittels eines Extruders zu Strängen extrudiert. Diese wurden in Abschnitte von 6 cm geschnitten und dann in einem Drehrohrofen bei 700 bis 900°C 2 h lang calziniert, anschließend auf eine mittlere Teilchengröße von etwa 5 μm gemahlen und mit 1 Gew.-% feinteiligem Siliciumdioxid (Rüttelgewicht 150 g/l, mittlere Teilchengröße 10 μm, BET-Oberfläche 100 m²/g) vermischt.

Die vorab hergestellte Promotorphase und die Wirtsphasen-Ausgangsmasse wurden unter Zumischung von 1,5 Gew.-% Graphit (laut Siebanalyse min. 50 Gew.-% < 24 μm; 24 μm < max. 10 Gew.-% < 48 μm; max. 5 Gew.-% >

48 μm; BET-Oberfläche 6-13 m$^2$/g) in einem Verhältnis gemischt, so dass man eine Masse der Stöchiometrie [Bi$_2$W$_2$O$_9$ x 2WO$_3$]$_{0,5}$[Mo$_{12}$Co$_{5,5}$Fe$_{2,94}$Si$_{1,59}$K$_{0,08}$O$_y$]$_1$ erhielt. Aus der Masse wurden zylindrische Ringe mit 5 mm Außendurchmesser, 3 mm Höhe und 2 mm Lochdurchmesser geformt.

Die geformten Ringe wurden in einer Schütthöhe von 50 bis 70 mm auf das Band einer Bandcalziniervorrichtung mit acht Kammern gelegt. Die Kammern wiesen jeweils einen Ventilator zur Erzeugung einer Luftzirkulation auf und waren auf 180 °C, 200 °C, 290 °C, 390 °C, 465 °C, 465 °C bzw. 435 °C thermostatiert. Innerhalb der Kammern war die zeitliche und örtliche Abweichung der Temperatur vom Sollwert stets ≤ 2 °C. Die Länge der Kammern war dergestalt, dass die Verweilzeit in den ersten vier Kammern jeweils 1,5 h und in der fünften bis achten Kammer jeweils 2 h betrug. Auf diese Weise wurden 2,6 t Katalysator hergestellt. Aus jedem Gebinde mit 100 kg Füllgewicht wurde jeweils eine Punktprobe von 1 kg Umfang entnommen und wie vorstehend beschrieben charakterisiert. Die erhaltenen Ergebnisse sind in Tabelle 1 aufgeführt.

Tabelle 1

| Laufende Nr. | Aktivität [°C] | $S_{ACR+ACS}$ [mol-%] |
|---|---|---|
| 1 | 315 | 92,7 |
| 2 | 317 | 93 |
| 3 | 317 | 92,3 |
| 4 | 317 | 92,3 |
| 5 | 319 | 92,6 |
| 6 | 319 | 93,6 |
| 7 | 320 | 94 |
| 8 | 322 | 94,7 |
| 9 | 316 | 93,7 |
| 10 | 319 | 94,1 |
| 11 | 321 | 94,1 |
| 12 | 320 | 92,8 |
| 13 | 322 | 93,6 |
| 14 | 321 | 94,9 |
| 15 | 320 | 92,9 |
| 16 | 317 | 93 |
| 17 | 317 | 92,6 |
| 18 | 320 | 93 |
| 19 | 320 | 92,7 |
| 20 | 317 | 92,9 |
| 21 | 318 | 93,9 |
| 22 | 320 | 93,2 |
| 23 | 318 | 92,7 |
| 24 | 322 | 93,9 |
| 25 | 320 | 92,3 |
| 26 | 317 | 94 |
| Mittelwert | 319 | 93,3 |
| Standardabweichung | 0,62 % | 0,80 % |

Vergleichsbeispiel 1

Der Katalysatorvorläufer aus Beispiel 1 wurde in Portionen von 500 g in einem üblichen Labormuffelofen, bei dem die zeitliche und örtliche Temperaturfluktuation etwa ± 3 °C beträgt, bei verschiedenen Solltemperaturen über die Dauer von 6 h calziniert. Die Katalysatoreigenschaften in Abhängigkeit von der Calzinierungstemperatur sind in Tabelle 2 angegeben.

Tabelle 2

| Versuch | $T_{calzination}$ [°C] | Aktivität [°C] | $S_{ACR+ACS}$ [mol-%] |
|---------|------------------------|----------------|------------------------|
| 1 | 443 | 314 | 84,5 |
| 2 | 446 | 311 | 92,3 |
| 3 | 449 | 309 | 93,6 |
| 4 | 460 | 321 | 94,7 |
| 5 | 467 | 329 | 96,2 |
| 6 | 471 | 330 | 96,7 |
| 7 | 473 | 330 | 96,6 |
| 8 | 480 | 377 | 96,3 |
| 9 | 480 | 343 | 96,9 |

Die Tabelle 2 zeigt, dass z. B. eine Temperaturdifferenz von 7 °C bei der Calzinierung (473 °C bzw. 480 °C) zu einem Aktivitätsunterschied von 47 °C (330 °C bzw. 377 °C) führen kann. Temperaturdifferenzen in dieser Größenordnung sind bei der Calzinierung großer Katalysatormengen nach herkömmlichen Verfahren, wie unter Verwendung von Hordenöfen, unvermeidlich. Die Versuche 8/9 zeigen, dass auch bei gleicher Solltemperatur unterschiedliche Ergebnisse erzielt werden, da die tatsächliche Temperatur ohne erzwungene Konvektion auch bei Portionen von 500 g sehr inhomogen ist.

Vergleichsbeispiel 2

300 kg des Katalysatorvorläufers aus Beispiel 1 wurden auf 9 übereinander angeordneten Horden eines Wagens verteilt. Der Wagen wurde in einen Ofen eingeschoben und im Luftstrom aufgeheizt. Man heizte innerhalb einer Stunde auf 185 °C, hielt die Temperatur 1 h auf diesem Wert, heizte dann mit einer Geschwindigkeit von 2 °C/min. auf 210 °C, hielt die Temperatur 2 h auf diesem Wert, heizte dann mit einer Geschwindigkeit von 2 °C/min. auf 250 °C, hielt die Temperatur 1 h auf diesem Wert, heizte dann mit einer Geschwindigkeit von 5 °C/min. auf 465 °C und hielt die Temperatur 6 h bei diesem Wert. Bei den angegebenen Temperaturen handelt es sich um die am Ofen eingestellten Solltemperaturen. Auf den einzelnen Horden des Wagens herrschten davon abweichende, unterschiedliche Temperaturen. Von jeder dieser Partien wurde eine Durchschnittsprobe aller Einzelbleche genommen und nachfolgend getestet.

Tabelle 3

| Partien | Aktivität [°C] | $S_{ACR+ACS}$ [mol-%] |
|---------|----------------|------------------------|
| 1 | 330 | 94,7 |
| 2 | 339 | 95,6 |
| 3 | 324 | 95,2 |
| 4 | 341 | 94,8 |
| 5 | 334 | 94,8 |
| 6 | 344 | 95,1 |
| 7 | 330 | 95 |
| 8 | 340 | 94,9 |
| 9 | 345 | 95,4 |

(fortgesetzt)

| Partien | Aktivität [°C] | $S_{ACR+ACS}$ [mol-%] |
|---|---|---|
| 10 | 332 | 94,6 |
| 11 | 332 | 94,2 |
| 12 | 346 | 96,1 |
| 13 | 350 | 96,2 |
| 14 | 332 | 96,3 |
| 15 | 331 | 95,3 |
| 16 | 330 | 94,8 |
| 17 | 330 | 95,1 |
| 18 | 323 | 95,6 |
| 19 | 345 | 95,6 |
| 20 | 335 | 95,6 |
| 21 | 328 | 94,8 |
| 22 | 334 | 95,8 |
| 23 | 329 | 95,5 |
| 24 | 328 | 96,8 |
| 25 | 334 | 95,3 |
| 26 | 342 | 96,5 |
| 27 | 331 | 94,7 |
| 28 | 336 | 94,9 |
| 29 | 326 | 95 |
| 30 | 338 | 95,1 |
| 31 | 326 | 94,2 |
| 32 | 331 | 95,3 |
| 33 | 322 | 94,6 |
| 34 | 330 | 95,6 |
| 35 | 345 | 95,4 |
| 36 | 323 | 96 |
| 37 | 333 | 95,7 |
| 38 | 337 | 95,7 |
| 39 | 340 | 95,8 |
| 40 | 322 | 94 |
| 41 | 343 | 95,1 |
| 42 | 334 | 94,5 |
| 43 | 335 | 95,1 |
| 44 | 336 | 94,4 |
| 45 | 345 | 95,2 |
| 46 | 338 | 95,6 |
| 47 | 343 | 95,3 |

(fortgesetzt)

| Partien | Aktivität [°C] | $S_{ACR+ACS}$ [mol-%] |
|---|---|---|
| 48 | 344 | 96,4 |
| 49 | 345 | 95,8 |
| 50 | 348 | 96,3 |
| 51 | 350 | 95,9 |
| 52 | 350 | 96,4 |
| 53 | 346 | 96,1 |
| 54 | 345 | 96,6 |
| 55 | 338 | 95,3 |
| 56 | 344 | 96,1 |
| 57 | 342 | 96,1 |
| 58 | 341 | 96,4 |
| 59 | 348 | 96 |
| 60 | 344 | 94,9 |
| 61 | 348 | 96,2 |
| 62 | 339 | 94,4 |
| 63 | 328 | 93,8 |
| 64 | 340 | 94,9 |
| 65 | 332 | 95,5 |
| 66 | 332 | 95 |
| 67 | 334 | 95,1 |
| 68 | 344 | 95,4 |
| 69 | 334 | 95,1 |
| 70 | 333 | 94,8 |
| 71 | 337 | 94,6 |
| 72 | 337 | 95,5 |
| 73 | 344 | 94,7 |
| 74 | 328 | 94,5 |
| 75 | 325 | 94,3 |
| 76 | 343 | 95,5 |
| 77 | 329 | 95,5 |
| 78 | 340 | 96,3 |
| 79 | 336 | 96 |
| 80 | 342 | 95,3 |
| 81 | 331 | 94,9 |
| 82 | 341 | 95,1 |
| 83 | 335 | 94,7 |
| 84 | 341 | 94,7 |
| 85 | 331 | 94,8 |

(fortgesetzt)

| Partien | Aktivität [°C] | $S_{ACR+ACS}$ [mol-%] |
|---|---|---|
| 86 | 329 | 95 |
| 87 | 324 | 94,4 |
| 88 | 333 | 95,6 |
| 89 | 333 | 94,1 |
| 90 | 334 | 94,2 |
| Mittelwert | 336,3 | 95,3 |
| Standardabweichung | 2,168 % | 0,703 % |

Beispiel 2

Es wurde zunächst eine Ausgangsmasse der Wirtsphase hergestellt. Hierzu wurden bei 60 °C portionsweise 213 kg Ammoniumheptamolybdat in 600 1 Wasser gelöst und unter Rühren mit 0,97 kg einer 46,8 gew.-%igen Kaliumhydroxidlösung von 20 °C versetzt. Es wurde eine zweite Lösung hergestellt, indem man zu 262,9 kg einer Cobaltnitratlösung (12,4 Gew.-% Cobalt) 80,2 kg einer Eisennitratlösung (14,2 Gew.-% Eisen) gab, wobei die Temperatur auf 60 °C gehalten wurde. Die zweite Lösung wurde innerhalb eines Zeitraums von 30 Min. bei 60 °C zu der Molybdatlösung gegeben. 15 Min. nach beendeter Zugabe wurden 19,16 kg Kieselsol (Dichte 1,36-1,42 g/ml; pH 8,5-9,5; Alkaligehalt max. 0,5 Gew.-%; 46,80 Gew.-% $SiO_2$) in die erhaltene Maische gegeben. Danach rührte man 15 Min. Die erhaltene Maische wurde anschließend sprühgetrocknet, wobei ein Pulver mit einem Glühverlust (3 h bei 600 °C) von etwa 30 Gew.-% erhalten wurde.

Die Herstellung der feinteiligen Promotorphase erfolgte wie in Beispiel 1.

Die vorab hergestellte Promotorphase und die Wirtsphasen-Ausgangsmasse wurden unter Zumischung von 1,5 Gew.-% Graphit in einem Verhältnis gemischt, so dass man eine Masse der Stöchiometrie $[Bi_2W_2O_9 \times 2WO_3]_{0,5}$ $[Mo_{12}Co_{5,5}Fe_{2,0}Si_{1,59}K_{0,08}O_y]_1$ erhielt. Aus der Masse wurden zylindrische Ringe mit nur 5 mm Außendurchmesser, 2 mm Höhe und 2 mm Lochdurchmesser geformt. Die geformten Ringe wurden wie in Beispiel 1 beschrieben calziniert. Die Charakterisierung des erhaltenen Katalysators erfolgte ebenfalls wie in Beispiel 1 beschrieben. Die erhaltenen Ergebnisse sind in Tabelle 4 aufgeführt.

Tabelle 4

| Laufende Nr. | Aktivität [°C] | $S_{ACR+ACS}$ [mol-%] |
|---|---|---|
| 1 | 320 | 93,7 |
| 2 | 316 | 92,6 |
| 3 | 322 | 93,1 |
| 4 | 322 | 92,9 |
| 5 | 321 | 92,3 |
| 6 | 319 | 94,0 |
| 7 | 320 | 92,5 |
| 8 | 317 | 92,1 |
| 9 | 320 | 92,5 |
| 10 | 318 | 92,8 |
| 11 | 320 | 92,7 |
| 12 | 318 | 92,2 |
| 13 | 321 | 93,9 |
| 14 | 318 | 93,5 |
| 15 | 322 | 93,9 |

(fortgesetzt)

| Laufende Nr. | Aktivität [°C] | $S_{ACR+ACS}$ [mol-%] |
|---|---|---|
| 16 | 324 | 93,8 |
| 17 | 317 | 92,5 |
| 18 | 318 | 92,9 |
| 19 | 322 | 93,9 |
| 20 | 321 | 92,7 |
| Mittelwert | 320 | 93,0 |
| Standardabweichung | 0,67 % | 0,69 % |

Vergleichsbeispiel 3

Der Katalysatorvorläufer aus Beispiel 2 wurde in Portionen von 500 g in einem üblichen Labormuffelofen, bei dem die zeitliche und örtliche Temperaturfluktuation etwa ± 3 °C beträgt, bei verschiedenen Solltemperaturen über die Dauer von 6 h calziniert. Die Katalysatoreigenschaften in Abhängigkeit von der Calzinierungstemperatur sind in Tabelle 5 angegeben.

Tabelle 5

| Versuch | $T_{calzination}$ [°C] | Aktivität [OC] | $S_{ACR+ACS}$ [mol-%] |
|---|---|---|---|
| 1 | 440 | 316 | 83 |
| 2 | 445 | 312 | 92,1 |
| 3 | 450 | 310 | 93,4 |
| 4 | 460 | 321 | 94,2 |
| 5 | 467 | 327 | 96,4 |
| 6 | 471 | 330 | 96,7 |
| 7 | 475 | 333 | 96,8 |
| 8 | 477 | 340 | 97,5 |
| 9 | 480 | 370 | 96,5 |

Beispiel 3

Bei 60 °C löste man portionsweise 213 kg Ammoniumheptamolybdat in 600 1 Wasser und gab unter Rühren 0,97 kg einer 46,8 gew.-%igen Kaliumhydroxidlösung von 20 °C dazu. Es wurde eine zweite Lösung hergestellt, indem man zu 333,7 kg einer Cobaltnitratlösung (12,4 Gew.-% Cobalt) 116,25 kg einer Eisennitratlösung (14,2 Gew.-% Eisen) gab, wobei die Temperatur auf 30 °C gehalten und nach beendeter Zugabe noch 30 Min. gerührt wurde. Bei 60°C dosierte man 112,3 kg einer Wismutnitratlösung (11,2 % Wismut) zu der Eisen-Cobalt-Lösung. Die zweite Lösung wurde innerhalb eines Zeitraums von 30 Min. bei 60 °C zu der Molybdatlösung gegeben. 15 Min. nach beendeter Zugabe wurden 19,16 kg Kieselsol (46,80 Gew.-% $SiO_2$) in die erhaltene Maische gegeben. Danach rührte man 15 Min. Die erhaltene Maische wurde anschließend sprühgetrocknet, wobei ein Pulver mit einem Glühverlust (3 h bei 600 °C) von etwa 30 Gew.-% erhalten wurde.

Die Zusammensetzung der Aktivmasse ist $Mo_{12}CO_7Fe_{2,94}Bi_{0,6}Si_{1,59}Ko_{0,08}O_z$.

Diese Ausgangsmasse wurde nach dem Sprühtrocknen mit 1,5 Gew.-% Graphit gemischt, kompaktiert und zu zylindrischen Ringen mit 5 mm Außendurchmesser, 3 mm Höhe und 2 mm Lochdurchmesser geformt. Die geformten Ringe wurden in einer Bandcalziniervorrichtung mit acht Kammern calziniert. Die Kammern waren auf 160 °C, 200 °C, 230 °C, 270 °C, 380 °C, 430 °C, 500 °C bzw. 500 °C thermostatiert. Die Verweilzeit betrug je 2 h in der ersten bis vierten Kammer und 5 h in der fünften bis achten Kammer. Die Charakterisierung des erhaltenen Katalysators erfolgte wie in Beispiel 1. Die erhaltenen Ergebnisse sind in Tabelle 6 aufgeführt.

Tabelle 6

| Laufende Nr. | Aktivität [°C] | $S_{ACR+ACS}$ [mol-%] |
|---|---|---|
| 1 | 319 | 95,6 |
| 2 | 318 | 95,7 |
| 3 | 317 | 95,7 |
| 4 | 317 | 95,3 |
| 5 | 319 | 95,5 |
| 6 | 320 | 95,4 |
| 7 | 319 | 95,7 |
| 8 | 319 | 95,4 |
| 9 | 319 | 95,4 |
| 10 | 318 | 95,4 |
| 11 | 319 | 95,4 |
| 12 | 321 | 95,8 |
| 13 | 320 | 95,8 |
| 14 | 320 | 95,7 |
| 15 | 323 | 96,0 |
| 16 | 318 | 95,8 |
| 17 | 321 | 96,1 |
| Mittelwert | 319 | 95,6 |
| Standardabweichung | 0,48 % | 0,24 % |

Vergleichsbeispiel 4

Der Katalysatorvorläufer aus Beispiel 3 wurde in Portionen von 500 g in einem üblichen Labormuffelofen, bei dem die zeitliche und örtliche Temperaturfluktuation etwa $\pm$ 3 °C beträgt, bei verschiedenen Solltemperaturen über die Dauer von 6 h calziniert.

Die Katalysatoreigenschaften in Abhängigkeit von der Calzinierungstemperatur sind in Tabelle 7 angegeben.

Tabelle 7

| Versuch | $T_{calzination}$ [°C] | Aktivität [°C] | $S_{ACR+ACS}$ [mol-%] |
|---|---|---|---|
| 1 | 461 | 309 | 94,3 |
| 2 | 462 | 307 | 95,2 |
| 3 | 466 | 313 | 94,3 |
| 4 | 467 | 317 | 95,1 |
| 5 | 469 | 313 | 95,4 |
| 6 | 471 | 316 | 96 |
| 7 | 479 | 324 | 96,7 |
| 8 | 485 | 326 | 96,5 |
| 9 | 491 | 324 | 96,9 |
| 10 | 493 | 331 | 97,3 |
| 11 | 500 | 347 | 97,4 |

Beispiel 4

Beispiel 1 wurde wiederholt, wobei jedoch eine Bandcalziniervorrichtung mit 12 Zonen verwendet wurde. Temperatur und Verweilzeit in den ersten acht Zonen waren wie in Beispiel 1 angegeben. Die Zonen 9 bis 12 waren einheitlich auf 500 °C thermostatiert, und die Verweilzeit betrug jeweils 2 h. Die Atmosphäre in den Kammern 9 bis 12 bestand im Wesentlichen aus Stickstoff. Auf diese Weise wurden 3,5 t Katalysator hergestellt. Aus jedem Gebinde mit 100 kg Füllgewicht wurde jeweils eine Punktprobe von 1 kg Umfang entnommen und wie vorstehend beschrieben charakterisiert. Die erhaltenen Ergebnisse sind in Tabelle 8 aufgeführt.

Tabelle 8

| Laufende Nr. | Aktivität [°C] | $S_{ACR+ACS}$ [mol-%] |
|---|---|---|
| 1 | 315 | 94,7 |
| 2 | 316 | 93 |
| 3 | 317 | 94,3 |
| 4 | 317 | 94,3 |
| 5 | 319 | 94,6 |
| 6 | 318 | 93,8 |
| 7 | 319 | 94,6 |
| 8 | 317 | 93,7 |
| 9 | 314 | 93,4 |
| 10 | 318 | 95 |
| 11 | 320 | 95,1 |
| 12 | 320 | 95,2 |
| 13 | 318 | 94,8 |
| 14 | 317 | 94,3 |
| 15 | 314 | 94 |
| 16 | 318 | 94,8 |
| 17 | 318 | 94,8 |
| 18 | 318 | 94,7 |
| 19 | 317 | 94,3 |
| 20 | 314 | 94 |
| 21 | 319 | 94,5 |
| 22 | 320 | 95 |
| 23 | 320 | 94,8 |
| 24 | 318 | 94,7 |
| 25 | 317 | 94,6 |
| 26 | 316 | 94,7 |
| 27 | 319 | 95 |
| 28 | 317 | 94,5 |
| 29 | 319 | 95 |
| 30 | 314 | 94,4 |
| 31 | 316 | 94,6 |
| 32 | 317 | 94,7 |

(fortgesetzt)

| Laufende Nr. | Aktivität [°C] | $S_{ACR+ACS}$ [mol-%] |
|---|---|---|
| 33 | 317 | 94,8 |
| 34 | 318 | 94,7 |
| 35 | 319 | 95 |
| Mittelwert | 317,4 | 94,5 |
| Standardabweichung | 0,552 % | 0,515 % |

Beispiel 5

In einem Mischer wurden nacheinander 191,5 kg $MoO_3$, 45,6 kg CoO, 27,2 kg $Fe_2O_3$, 10,4 kg $SiO_2$, 0,4 kg $K_2O$ innig vermischt; anschlie-ßend wurde die in Beispiel 1 hergestellte Promotorphase in einem Verhältnis zugegeben, so dass man eine Masse der Stöchiometrie $[Bi_2W_2O_9x2WO_3]_{0,5}[Mo_{12}Co_{5,5}Fe_{3,0}Si_{1,6}K_{0,08}O_y]$ erhielt, die zu zylindrischen Ringen mit 5 mm Außendurchmesser, 2 mm Höhe und 2 mm Lochdurchmesser geformt wurde. Die geformten Ringe wurden in einer Bandcalziniervorrichtung calziniert, die drei Kammern aufwies, die auf 270 °C, 465 °C bzw. 465 °C thermostatiert waren. Die Verweilzeit pro Kammer betrug 3 h. Auf diese Weise wurden 2,5 t Katalysator hergestellt. Aus jedem Gebinde mit 100 kg Füllgewicht wurde jeweils eine Punktprobe von 1 kg Umfang entnommen und wie vorstehend beschrieben charakterisiert. Die erhaltenen Ergebnisse sind in Tabelle 9 aufgeführt.

Tabelle 9

| Laufende Nr. | Aktivität [°C] | $S_{ACR+ACS}$ [mol-%] |
|---|---|---|
| 1 | 317 | 93,7 |
| 2 | 318 | 94 |
| 3 | 317 | 94,3 |
| 4 | 319 | 95 |
| 5 | 317 | 94 |
| 6 | 320 | 94,5 |
| 7 | 320 | 94 |
| 8 | 321 | 94,8 |
| 9 | 317 | 93 |
| 10 | 316 | 93 |
| 11 | 317 | 93,4 |
| 12 | 319 | 93,8 |
| 13 | 315 | 93 |
| 14 | 317 | 93,6 |
| 15 | 318 | 93,8 |
| 16 | 319 | 93,8 |
| 17 | 319 | 94 |
| 18 | 318 | 93,5 |
| 19 | 317 | 93,4 |
| 20 | 315 | 93,8 |
| 21 | 319 | 93 |
| 22 | 316 | 93,6 |
| 23 | 320 | 93,8 |

(fortgesetzt)

| Laufende Nr. | Aktivität [°C] | $S_{ACR+ACS}$ [mol-%] |
|---|---|---|
| 24 | 318 | 93 |
| 25 | 317 | 92,8 |
| Mittelwert | 317,8 | 93,7 |
| Standardabweichung | 0,495 % | 0,609 % |

Beispiel 6

Beispiel 3 wurde wiederholt, wobei jedoch die Mengen der Komponenten so gewählt wurden, dass die chemische Zusammensetzung $Mo_{12}Co_7Fe_3Bi_{1,0}Si_{1,6}K_{0,08}O_z$ erhalten wurde. Die Calzinierung erfolgte in einer Bandcalziniervorrichtung mit acht Zonen, die auf 160 °C, 210 °C, 240 °C, 290 °C, 380 °C, 515 °C, 515 °C bzw. 400 °C thermostatiert waren. Aus jedem Gebinde mit 100 kg Füllgewicht wurde jeweils eine Punktprobe von 1 kg Umfang entnommen und wie vorstehend beschrieben charakterisiert. Die erhaltenen Ergebnisse sind in Tabelle 10 aufgeführt.

Tabelle 10

| Laufende Nr. | Aktivität [°C] | $S_{ACR+ACS}$ [mol-%] |
|---|---|---|
| 1 | 317 | 94,9 |
| 2 | 318 | 95,8 |
| 3 | 319 | 94,7 |
| 4 | 317 | 95,1 |
| 5 | 319 | 95,2 |
| 6 | 320 | 94,8 |
| 7 | 321 | 96 |
| 8 | 318 | 94,8 |
| 9 | 319 | 94,8 |
| 10 | 317 | 95,7 |
| 11 | 316 | 95 |
| 12 | 318 | 95,6 |
| 13 | 318 | 95 |
| 14 | 319 | 95,7 |
| 15 | 317 | 94,9 |
| 16 | 319 | 95,3 |
| 17 | 318 | 95 |
| 18 | 317 | 94,7 |
| 19 | 318 | 95 |
| 20 | 319 | 95,6 |
| 21 | 319 | 95,7 |
| 22 | 320 | 95,7 |
| 23 | 320 | 95,8 |
| 24 | 318 | 95 |
| 25 | 317 | 94,8 |
| Mittelwert | 318,3 | 95,2 |

(fortgesetzt)

| Laufende Nr. | Aktivität [°C] | $S_{ACR+ACS}$ [mol-%] |
|---|---|---|
| Standardabweichung | 0,382 % | 0,440 % |

Beispiel 7

In einem Mischer wurden nacheinander 114,4 kg $MoO_3$, 34,5 kg CoO, 15,8 kg $Fe_2O_3$, 6,3 kg $SiO_2$, 9,2 kg $Bi_2O_3$ und 0,2 kg $K_2O$ homogen vermischt. Das Gemisch wurde zu zylindrischen Ringen mit 5 mm Au-ßendurchmesser, 3 mm Höhe und 2 mm Lochdurchmesser geformt.

Die geformten Ringe wurden in einer Bandcalziniervorrichtung mit drei Kammern calziniert, die auf 350 °C, 510 °C bzw. 510 °C thermostatiert waren. Die Verweilzeit pro Kammer betrug 2,5 h. Auf diese Weise wurden 2,5 t Katalysator hergestellt. Aus jedem Gebinde mit 100 kg Füllgewicht wurde jeweils eine Punktprobe von 1 kg Umfang entnommen und wie vorstehend beschrieben charakterisiert. Die erhaltenen Ergebnisse sind in Tabelle 11 aufgeführt.

Tabelle 11

| Laufende Nr. | Aktivität [°C] | $S_{ACR+ACS}$ [mol-%] |
|---|---|---|
| 1 | 316 | 94,9 |
| 2 | 318 | 95,8 |
| 3 | 317 | 94,7 |
| 4 | 319 | 95,1 |
| 5 | 316 | 95,2 |
| 6 | 320 | 94,8 |
| 7 | 318 | 96 |
| 8 | 321 | 94,8 |
| 9 | 318 | 94,8 |
| 10 | 315 | 95,7 |
| 11 | 317 | 95 |
| 12 | 319 | 95,6 |
| 13 | 315 | 95 |
| 14 | 318 | 95,7 |
| 15 | 318 | 94,9 |
| 16 | 319 | 95,3 |
| 17 | 319 | 95 |
| 18 | 320 | 94,7 |
| 19 | 317 | 95 |
| 20 | 315 | 95,6 |
| 21 | 319 | 95,7 |
| 22 | 315 | 95,7 |
| 23 | 320 | 95,8 |
| 24 | 318 | 95 |
| 25 | 316 | 94,8 |
| Mittelwert | 317,7 | 95,2 |
| Standardabweichung | 0,557 % | 0,440 % |

**Patentansprüche**

1. Verfahren zur Herstellung eines für die Gasphasenoxidation von organischen Verbindungen zu $\alpha,\beta$-ungesättigten Aldehyden und/oder Carbonsäuren geeigneten Katalysators mit einer aktiven Phase aus einer Multimetalloxidmasse, bei dem man einen teilchenförmigen Katalysatorvorläufer herstellt, der Oxide und/oder in Oxide überführbare Verbindungen der von Sauerstoff verschiedenen, die Multimetalloxidmasse konstituierenden Elemente enthält, und diesen durch Calzinierung in eine katalytisch aktive Form überführt, **dadurch gekennzeichnet, dass** man einen Strom des teilchenförmigen Katalysatorvorläufers zur Calzinierung mit im Wesentlichen konstanter Geschwindigkeit durch wenigstens eine Calzinierungszone führt, und senkrecht zur Fortbewegungsrichtung des Katalysatorvorläufers in der Calzinierungszone einen Gasstrom durch den Strom des Katalysatorvorläufers leitet, wobei die maximale zeitliche Schwankung der Temperatur und die maximale lokale Temperaturdifferenz in der Calzinierungszone jeweils $\leq 5\ °C$ betragen.

2. Verfahren nach Anspruch 1, **dadurch gekennzeichnet, dass** die Multimetalloxidmasse wenigstens ein unter Molybdän und Wolfram ausgewähltes erstes Metall und wenigstens ein unter Wismut, Tellur, Antimon, Zinn, Kupfer, Eisen, Cobalt und/oder Nickel ausgewähltes zweites Metall enthält.

3. Verfahren nach Anspruch 2, **dadurch gekennzeichnet, dass** die Multimetalloxidmasse die Formel I oder II aufweist,

$$[X^1_a X^2_b O_x]_p [X^3_c X^4_d X^5_e X^6_f X^7_g X^2_h O_y]_q \qquad (I)$$

$$Mo_{12} Bi_i X^8_k Fe_1 X^9_m X^{10}_n O_z \qquad (II)$$

worin bedeuten

$X^1$ Wismut, Tellur, Antimon, Zinn und/oder Kupfer,
$X^2$ Molybdän und/oder Wolfram,
$X^3$ ein Alkalimetall, Thallium und/oder Samarium,
$X^4$ ein Erdalkalimetall, Nickel, Cobalt, Kupfer, Mangan, Zink, Zinn, Cadmium und/oder Quecksilber,
$X^5$ Eisen, Chrom, Cer und/oder Vanadium,
$X^6$ Phosphor, Arsen, Bor und/oder Antimon,
$X^7$ ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
a 0,01 bis 8,
b 0,1 bis 30,
c 0 bis 4,
d 0 bis 20,
e 0 bis 20,
f 0 bis 6,
g 0 bis 15,
h 8 bis 16,
x,y Zahlen, die durch die Wertigkeit und Häufigkeit der von Sauerstoff verschiedenen Elemente in I bestimmt sind,
p,q Zahlen, deren Verhältnis p/q 0,1 bis 10 beträgt,
$X^8$ Cobalt und/oder Nickel,
$X^9$ Silicium und/oder Aluminium,
$X^{10}$ ein Alkalimetall,
i 0,1 bis 2,
k 2 bis 10,
l 0,5 bis 10,
m 0 bis 10,
n 0 bis 0,5,
z Zahl, die durch die Wertigkeit und Häufigkeit der von Sauerstoff verschiedenen Elemente in II bestimmt ist.

4. Verfahren nach einem der vorhergehenden Ansprüche, bei dem man den Katalysatorvorläufer durch wenigstens zwei Calzinierungszonen führte die auf unterschiedliche Temperatur thermostatisiert sind.

5. Verfahren nach einem der vorhergehenden Ansprüche, bei dem die maximale zeitliche Schwankung der Temperatur in der Calzinierungszone $\leq 3\ °C$ beträgt.

**6.** Verfahren nach Anspruch 5, bei dem die maximale zeitliche Schwankung der Temperatur in der Calzinierungszone ≤ 2 °C beträgt.

**7.** Verfahren nach einem der vorhergehenden Ansprüche, bei dem die maximale lokale Temperaturdifferenz in der Calzinierungszone ≤ 3 °C beträgt.

**8.** Verfahren nach Anspruch 7, bei dem die maximale lokale Temperaturdifferenz in der Calzinierungszone ≤ 2 °C beträgt.

**Claims**

**1.** A process for the preparation of a catalyst suitable for the gas phase oxidation of organic compounds to α,β-unsaturated aldehydes and/or carboxylic acids and having an active phase of a multimetal oxide material, in which a particulate catalyst precursor which comprises oxides and/or compounds of the elements other than oxygen which constitute the multimetal oxide material, which compounds can be converted into oxides, is prepared and said catalyst precursor is converted by calcination into a catalytically active form, wherein a stream of the particulate catalyst precursor is passed at substantially constant speed through at least one calcination zone for calcination, and a gas stream is passed through the catalyst precursor stream perpendicularly to the direction of advance of the catalyst precursor in the calcination zone, the maximum variation of the temperature as a function of time and the maximum local temperature difference in the calcination zone each being ≤ 5°C.

**2.** The process according to claim 1, wherein the multimetal oxide material comprises at least one first metal selected from molybdenum and tungsten and at least one second metal selected from bismuth, tellurium, antimony, tin, copper, iron, cobalt and/or nickel.

**3.** The process according to claim 2, wherein the multimetal oxide material has the formula I or II,

$$[X^1_a X^2_b O_x]_p [X^3_c X^4_d X^5_e X^6_f X^7_g X^2_h O_y]_q \qquad (I)$$

$$Mo_{12} Bi_i X^8_k Fe_1 X^9_m X^{10}_n O_z \qquad (II)$$

where

$X^1$ is bismuth, tellurium, antimony, tin and/or copper,
$X^2$ is molybdenum and/or tungsten,
$X^3$ is an alkali metal, thallium and/or samarium,
$X^4$ is an alkaline earth metal, nickel, cobalt, copper, manganese, zinc, tin, cadmium and/or mercury,
$X^5$ is iron, chromium, cerium and/or vanadium,
$X^6$ is phosphorus, arsenic, boron and/or antimony,
$X^7$ is a rare earth metal, titanium, zirconium, niobium, tantalum, rhenium, ruthenium, rhodium, silver, gold, aluminum, gallium, indium, silicon, germanium, lead, thorium and/or uranium,
a is from 0.01 to 8,
b is from 0.1 to 30,
c is from 0 to 4,
d is from 0 to 20,
e is from 0 to 20,
f is from 0 to 6,
g is from 0 to 15,
h is from 8 to 16,
x and y are numbers which are determined by the valency and frequency of the elements other than oxygen in I,
p and q are numbers whose ratio p/q is from 0.1 to 10,
$X^8$ is cobalt and/or nickel,
$X^9$ is silicon and/or aluminum,
$X^{10}$ is an alkali metal,
i is from 0.1 to 2,
k is from 2 to 10,
l is from 0.5 to 10,

m is from 0 to 10,

n is from 0 to 0.5,

z is a number which is determined by the valency and frequency of the elements other than oxygen in II.

4. The process according to any of the preceding claims, in which the catalyst precursor is passed through at least two calcination zones which are thermostated at different temperatures.

5. The process according to any of the preceding claims, in which the maximum variation of the temperature in the calcination zone as a function of time is $\leq 3°C$.

6. The process according to claim 5, in which the maximum variation of the temperature in the calcination zone as a function of time is $\leq 2°C$.

7. The process according to any of the preceding claims, in which the maximum local temperature difference in the calcination zone is $\leq 3°C$

8. The process according to claim 7, in which the maximum local temperature difference in the calcination zone is $\leq 2°C$.

**Revendications**

1. Procédé de préparation d'un catalyseur approprié pour l'oxydation en phase gazeuse de composés organiques ayant une phase active constituée d'une masse d'oxyde multimétallique pour donner des aldéhydes $\alpha,\beta$-insaturés et/ou des acides carboxyliques, dans lequel en prépare un précurseur de catalyseur sous forme de particules, qui contient des oxydes, et/ou des composés des éléments constitutifs de la masse d'oxyde multimétallique différents de l'oxygène, lesdits composés pouvant être convertis en oxydes, puis on le convertit par calcination en une forme catalytiquement active, **caractérisé en ce qu'**un courant du précurseur de catalyseur sous forme de particules est amené pour calcination avec une vitesse sensiblement constante à travers au moins une zone de calcination et un courant de gaz est dirigé à travers le courant du précurseur de catalyseur perpendiculairement au sens de déplacement du précurseur de catalyseur dans la zone de calcination, la fluctuation temporelle maximale de la température et la différence locale maximale de température dans la zone de calcination étant respectivement $\leq 5°C$.

2. Procédé selon la revendication 1, **caractérisé en ce que** la masse d'oxyde multimétallique contient au moins un premier métal sélectionné parmi le molybdène et le tungstène et au moins un deuxième métal sélectionné parmi le bismuth, le tellure, l'antimoine, l'étain, le cuivre, le fer, le cobalt et/ou le nickel.

3. Procédé selon la revendication 2, **caractérisé en ce que** la masse d'oxyde multimétallique correspond à la formule I ou II :

$$[X^1_a X^2_b O_x]_p [X^3_c X^4_d X^5_e X^6_f X^7_g X^2_h O_y]_q \qquad \text{(I)}$$

$$Mo_{12} Bi_i X^8_k Fe_l X^9_m X^{10}_n O_z \qquad \text{(II)}$$

dans lesquelles :

$X^1$ signifie bismuth, tellure, antimoine, étain et/ou cuivre,

$X^2$ molybdène et/ou tungstène,

$X^3$ un métal alcalin, thallium et/ou samarium,

$X^4$ un métal alcalino-terreux, nickel, cobalt, cuivre, manganèse, zinc, étain, cadmium et/ou mercure,

$X^5$ fer, chrome, cérium et/ou vanadium,

$X^6$ phosphore, arsenic, bore et/ou antimoine,

$X^7$ métal des terres rares, titane, zirconium, niobium, tantale, rhénium, ruthénium, rhodium, argent, or, aluminium, gallium, indium, silicium, germanium, plomb, thorium et/ou uranium,

a 0,01 à 8,

b 0,1 à 30,

c 0 à 4,

d 0 à 20,

e 0 à 20,

f 0 à 6,

g 0 à 15,

h 8 à 16,

x, y des nombres qui sont déterminés grâce à la valence et à la fréquence des éléments différents de l'oxygène dans I,

p, q des nombres, dont le rapport p/q est de 0,1 à 10,

$X^8$ cobalt et/ou nickel,

$X^9$ silicium et/ou aluminium,

$X^{10}$ un métal alcalin,

i 0,1 à 2,

k 2 à 10,

l 0,5 à 10,

m 0 à 10,

n 0 à 0,5,

z un nombre qui est déterminé grâce à la valence et à la fréquence des éléments différents de l'oxygène dans II.

**4.** Procédé selon l'une quelconque des revendications précédentes, dans lequel le précurseur de catalyseur est amené à travers au moins deux zones de calcination qui sont thermostatisées à une température différente.

**5.** Procédé selon l'une quelconque des revendications précédentes, dans lequel la fluctuation temporelle maximale de la température dans la zone de calcination est $\leq 3°C$.

**6.** Procédé selon la revendication 5, dans lequel la fluctuation temporelle maximale de la température dans la zone de calcination est $\leq 2°C$.

**7.** Procédé selon l'une quelconque des revendications précédentes, dans lequel la différence locale maximale de température dans la zone de calcination est $\leq 3°C$.

**8.** Procédé selon la revendication 7, dans lequel la différence locale maximale de température dans la zone de calcination est $\leq 2°C$.

Fig. 1

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5276178 A
- DE 3006894
- WO 9824746 A
- US 5198581 A
- US 4438217 A
- EP 0807465 A
- JP 08086571 A

- US 5637546 A
- EP 750942 A
- EP 0000835 A
- EP 0575897 A
- DE 19855913
- EP 558028 A